# EUROPÄISCHE PATENTSCHRIFT

(11) **EP 1 209 988 B1**
(45) Veröffentlichungstag und Bekanntmachung des Hinweises auf die Patenterteilung: **03.05.2006**
(21) Anmeldenummer: 00965920.2
(22) Anmeldetag: 05.09.2000
(51) Int. Cl.: A23L 1/308, A61K 9/00

(54) **VERNETZTES MITTEL ZUR ERZEUGUNG EINES LANGANHALTENDEN SÄTTIGUNGSEFFEKTS UND VERFAHREN ZU DESSEN HERSTELLUNG**
CROSS-LINKED AGENT FOR GENERATION OF A LONG-LASTING SATIETY EFFECT AND METHOD FOR THE PRODUCTION OF THE SAID
SUBSTANCE RETICULEE SERVANT A PRODUIRE UN EFFET RASSASIANT DURABLE ET PROCEDE DE PRODUCTION CORRESPONDANT

(30) Priorität: 06.09.1999 DE 19942417; 06.09.1999 DE 29915656 U
(43) Veröffentlichungstag der Anmeldung: 05.06.2002
(73) Patentinhaber: Beisel, Günther, 40789 Monheim (DE)
(72) Erfinder: Beisel, Günther, 40789 Monheim (DE)
(74) Vertreter: Fitzner, Ulrich
(86) Internationale Anmeldenummer: PCT/EP2000/008646
(87) Internationale Veröffentlichungsnummer: WO 2001/017377

(56) Entgegenhaltungen:
- EP-A- 0 792 653
- EP-A- 0 888 776
- EP-A- 0 901 792
- WO-A-98/23259
- DE-U- 29 915 634
- DE-U- 29 915 668
- SHAPIRO L ET AL: "NOVEL ALGINATE SPONGES FOR CELL CULTUREE AND TRANSPLANTATION" BIOMATERIALS,GB,ELSEVIER SCIENCE PUBLISHERS BV., BARKING, Bd. 18, Nr. 8, 1. April 1997 (1997-04-01), Seiten 583-590, XP000686398 ISSN: 0142-9612 in der Anmeldung erwähnt

## Beschreibung

Die vorliegende Erfindung betrifft ein Mittel zur Erzeugung eines Sättigungseffekts.

Es sind zahlreiche Versuche unternommen worden, auf medikamentösem Weg überflüssige Fettanreicherungen im menschlichen Körper abzubauen beziehungsweise deren Entstehung zu verhindern. Es gibt z.B. sogenannte Appetitzügler, die den Körper auf biochemischem Weg eine Abneigung zur Nahrungsaufnahme zu suggerieren versuchen. Diese Mittel haben zum Teil erhebliche schädliche Nebenwirkungen.

Neben den zahlreichen bekannten Diätvorschlägen gibt es auch mechanische und elektromechanische Mittel, mit denen ein gezielter Fettabbau beziehungsweise Muskelaufbau erfolgen soll. Die Wirkung solcher Mittel ist jedoch sehr zweifelhaft.

Aus der WO 98/23259 A sind schwammartige Gebilde zur oralen Applikation, enthaltend z. B. Alginate zur oralen Einnahme bekannt. Aktive Wirkstoffe können zusätzlich ebenfalls enthalten sein. Die Zusammensetzungen werden zur Erzielung eines Sättigungseffektes verwendet.

Aus der DE 4025912 ist ein Mittel zur oralen Einnahme bekannt, das aus einem im Magen lösbaren und den Inhalt freigebenden Behälter besteht. Dieser ist mit einem Stoff gefüllt, der nach seinem Freisetzen im Magen sein Volumen vergrößert und dadurch dem Körper ein Sättigungsgefühl suggeriert.

Aus dem Stand der Technik sind bereits eine Reihe von elastischen Materialien bekannt, die beim Durchtritt durch die Speiseröhre komprimierbar sind und die nach Verlassen der Speiseröhre in Wasser und/oder gastrointestinaler Flüssigkeit dekomprimierbar sind. Unter solchen schwammartigen Gebilden sind Schäume zu verstehen, die aus gasgefüllten, kugel-polyederförmigen Zellen bestehen, welche durch hochviskose oder feste Zellstege begrenzt sind. Einsetzbar sind erfindungsgemäß sowohl natürlich vorkommende Schwämme als auch synthetisch hergestellte schwammartige Gebilde.

Als natürliche Materialien finden Collagen und Cellulose bereits Anwendung. Allerdings handelt es sich bei den zuerst genannten Materialien um relativ teure Rohstoffe. Für beide Stoffe sind aufwendige Isolierungs- oder Aufarbeitungsverfahren notwendig, die zudem sehr umweltbelastend sind. Letzteres gilt vor allem für Cellulose, bei deren Isolierung große Mengen an Säuren aufgewendet werden müssen.

Lösliches Collagen beispielsweise wird aus Tierhäuten, vorzugsweise junger Rinder oder Schweine isoliert, da der Anteil an löslichem Collagen im Organismus mit zunehmendem After immer geringer wird. Dies ist ebenfalls nur mit aufwendigen Isolierungs- und Aufarbeitungsverfahren möglich.

Nicht zuletzt seit dem Bekanntwerden diverser und mutmaßlich auf den Menschen übertragbarer Seuchen bei Schwein und Rind, insbesondere der Rinderseuche BSE, und einem möglichen Infektionsrisiko für den Menschen, ist die Akzeptanz solcher Collagen-haltigen Produkte beim Endverbraucher drastisch gesunken.

Aufgabe der vorliegenden Erfindung ist es deshalb ein Material zur Herstellung eines Mittels zur Erzeugung eines langanhaltenden Sättigungseffektes zur Verfügung zu stellen, daß die zuvor genannten Nachteile nicht aufweist.

Dies wird erfindungsgemäß durch ein Mittel zur oralen Einnahme, enthaltend stabil miteinander vemetzte Uronsäure-haltige Polysaccharide in Form eines schwammartigen Gebildes gelöst, das in Wasser und/oder gastrointestinalen Flüssigkeiten schwer löslich bzw. gering resorbierbar ist und das sich dadurch auszeichnet, dass die Uronsäure-haltigen Polysaccharide durch ionische Bindungen miteinander vernetzt und zusätzlich durch kovalente Bindungen stabil miteinander quervemetzt sind. Besonders bevorzugte Polyuronsäure-haltige Polysaccharide sind Alginsäuren und deren Salze (Alginate). Aber auch niederveresterte Pectine, Xanthan, Tragant, Chondroitinsulfat sowie alle anderen Uronsäure-haltigen Verbindungen können erfindungsgemäß zum Einsatz kommen.

Alginsäure ist eine lineare Polyuronsäure aus wechselnden Anteilen von D-Mannuronsäure und L-Guluronsäure, die durch β-glykosidische Bindungen miteinander verknüpft sind, wobei die Carboxylgruppen nicht verestert sind. Ein Molekül Alginsäure kann sich aus etwa 150-1050 Uronsäure-Einheiten zusammensetzen, wobei das durchschnittliche Molekulargewicht in einem Bereich von 30-200 kDa variieren kann.

Das Polysaccharid Alginsäure ist ein Bestandteil der Zellwände von Braunalgen. Der Anteil der Alginsäure an der Trockenmasse der Algen kann hierbei bis zu 40% ausmachen. Die Gewinnung der Alginsäure erfolgt durch alkalische Extraktion mit an sich bekannten Methoden gemäß dem Stand der Technik. Die resultierende pulverförmige Alginsäure ist somit rein pflanzlich und weist eine hohe Biokompatibilität auf. Sie kann unter Bildung hochviskoser Lösungen die 300-fache Menge ihres Eigengewichtes an Wasser aufnehmen. In Gegenwart von mehrwertigen Kationen bildet Alginsäure sogenannte Gele. Die Bildung von Alginatgelen in Gegenwart zweiwertiger Kationen, wie Calcium oder Barium, sind bei Shapiro I., et al. (Biomaterials, 1997, 18: 583-90) beschrieben. Letzteres ist aufgrund seiner Toxizität für den Einsatz in Biomedizin jedoch nicht geeignet. Neben Calcium-Chlorid liefert auch Calcium-Glukonat geeignete zweiwertige Kationen. Generell sind alle physiologisch unbedenklichen Poly-Kationen, insbesondere zweiwertige Kationen verwendbar.

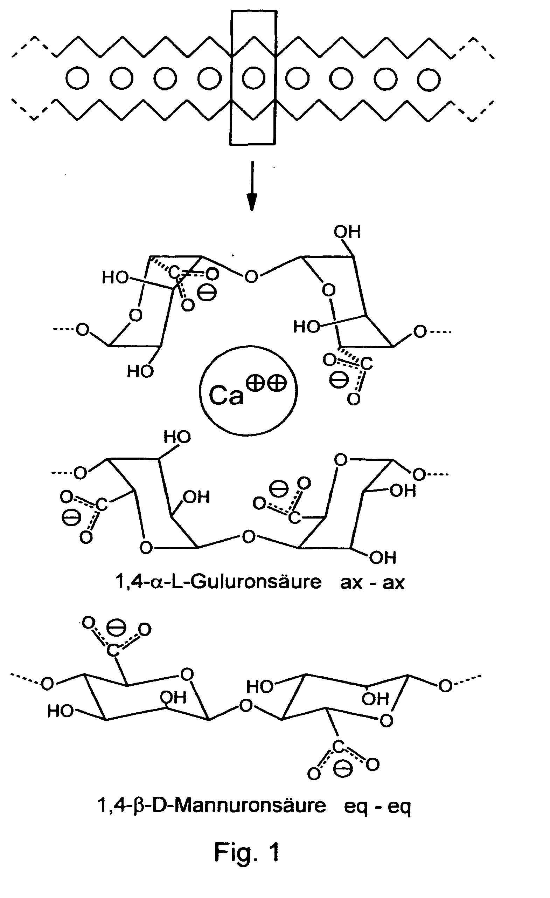

Die linearen, Ziehharmonika-ähnlichen Alginatketten werden durch die freien Bindungsstellen der Kationen, vorzugsweise Calcium-lonen, über ionische Bindungen fixiert (Fig. 1). Dadurch entsteht ein dreidimensionales Netzwerk, bei dem die zweiwertigen Kationen, wie in dem bei Smidsrod, et al. (Trends in Biotechnology, 1990, 8: 71) dargestellten "Egg-Box-Modell", wie "Eier in einem Eierkarton" liegen.

Die Herstellung der schwammartigen bzw. -förmigen Gebilde erfolgt mit an sich bekannten Methoden nach dem Stand der Technik. In Abhängigkeit von dem eingesetzten Ausgangsmaterial kann im einfachsten Falle ein Schaum durch Einblasen, durch Schlagen, Schütteln, Verspritzen oder Rühren in der betreffenden Gasatmosphäre erhalten werden. Bei den Polymeren entsteht die Schaumstruktur aufgrund chemischer Reaktionen. So werden z. B. Polyurethane durch Zugabe von Blähmitteln, die sich bei bestimmter Temperatur während der Verarbeitung unter Gasbildung zersetzen, oder durch Zusatz von flüssigen Lösemitteln während der Polymerisation, aufgeschäumt. Die Verschäumung erfolgt entweder beim Verlassen des Extrusionswerkzeuges, d.h. im Anschluß an das Extrudieren oder Spritzgießen oder in offenen Formen. Die Härtung erfolgt unter den für die jeweilige chemische Verbindung des Materials charakteristischen Bedingungen.

Unabdingbare Voraussetzung für die Einsetzbarkeit des Materials ist, daß es komprimierbar ist, ohne daß die Zellstege brechen. Um das erfindungsgemäße Material nämlich für die orale Einnahme einsetzen zu können, muß sich das schaumförmige bzw. schaumartige Material beim Durchtritt durch die Speiseröhre ohne weiteres komprimieren lassen. Insbesondere darf es beim Passieren der Speiseröhre nicht zu Beschwerden kommen.

Ein besonderer Vorteil der vorliegenden Erfindung ist, daß die erfindungsgemäß vemetzten Alginate flexibler und weicher sind und dadurch sehr viel günstigere mechanische Eigenschaften für die gastrointestinale Applikation aufweisen als die bisher auf dem Markt verfügbaren Materialien. Dies bringt für den Anwender den Vorteil einer besseren Verträglichkeit mit sich, so daß auch bei Patienten mit Schleimhautläsionen weder ein Druckgefühl noch Schleimhautreizungen hervorgerufen werden.

Für die Auswahl des Materials und die Art der Schaumbildung ist ferner wesentlich, daß es quellfähig bleibt, ohne daß die Zellstege zerstört werden. Nach dem Durchtritt durch die Speiseröhre soll das schwammartige Gebilde wenigstens wieder die Größe annehmen, die es vor dem Eintritt in die Speiseröhre hatte. Gegebenenfalls kann das Material auch zu einer Größe quellen, die über die ursprünglichen Volumina hinausgeht.

Das schwammartige Gebilde kann im komprimierten und dekomprimierten Zustand jede beliebige Form und Größe haben. Bevorzugt sind jedoch quaderförmige oder rechtecksförmige oder runde Ausgestaltungen.

Vorzugsweise ist das Material so ausgelegt, daß das schwammartige Gebilde auf 1/2 bis 1/100, vorzugsweise 1/4 bis 1/50, besonders bevorzugt 1/10 bis 1/20 seines Volumens bzw. seiner Größe komprimierbar ist. Unter physiologischen Bedingungen soll das komprimierte Material sich nach der Passage durch die Speiseröhre vorzugsweise auf das Zwei- bis Hundertfache, besonders bevorzugt auf das Vier- bis Fünfzigfache und ganz besonders bevorzugt auf das Zehnbis Zwanzigfache seines Volumens ausdehnen können.

Als Material für das schwammartige Gebilde können erfindungsgemäß natürliche, halbsynthetische oder synthetische Polymere zum Einsatz kommen, die ferner durch stabile Querverbindungen vernetzt sind.

Aus dem Stand der Technik sind verschiedene Verfahren zur Vernetzung von Polymeren bekannt. So ist beispielsweise die radikalische Polymerisation von Lactose-O-(p-Vinylbenzyl-)oxim zur Ausbildung von Hydrogelen bei Zhou, W-Z, et al. (Macromolecules, 1997, 30: 7063-7068) sowie eine Polymerisation N-Vinylpyrrolidon durch Elektronenstrahlung bei Rosiak, J.M. (J Contr Rel., 1994, 31: 9-19) beschrieben. Ferner sind beispielhaft vemetzte Polymere aus Saccharidacrylaten oder Poly(2-hydroxyethylmethacrylat-)Gelatine sowie Kollagen oder Chitosan bekannt (Martin, B.D., et al. (Biomaterials, 1998, 19: 69-76; Santin, M., et al. (Biomaterials, 1996, 17: 1459-1467); Weadock, K.S., et al. (J Biomed Mater Res, 1995, 29: 1371-1379); Groboillot, A.F., et al. (Biotech Bioeng, 1993, 42: 1157-1163)).

Beispiele für erfindungsgemäß besonders geeignete Ausgangsmaterialien sind Uronsäure-haltige Polysaccharide, die noch freie reaktive Gruppen, vorzugsweise Carboxylgruppen und/oder Hydroxylgruppen, zur Ausbildung stabiler Querverbindungen, wie z.B. Esterbindungen, aufweisen. Höchst bevorzugt sind hier Alginsäuren, niederveresterte Pectine, Xanthan, Tragant, Chondroitinsulfat sowie alle Uronsäure-haltigen Verbindungen und deren Salze.

Die Vernetzung von Alginaten durch mehrwertige Kationen ist bei Shapiro L et al., Biomaterials, 1997, 18:583-590 beschrieben. Diese Verbindungen sind jedoch in Wasser oder umgebendem Medium mit einer Calciumkonzentration unterhalb von 3 mmolar instabil, da sich das Calcium aus dem Kettenverband herauslöst und/oder gegebenenfalls durch andere (einwertige) Ionen verdrängt wird. Dies führt zu einer Auflösung der Vernetzung zwischen den Ziehharmonika-ähnlichen Polyuronsäure-haltigen Polysaccharidketten. Nachteilig ist hier, daß sich die nur durch ionische Bindungen vernetzten Alginate in Wasser und/oder gastrointestinalen Flüssigkeiten relativ schnell auflösen und somit nicht zur Erzeugung eines Sättigungseffekts geeignet sind. Ein besonderer Vorteil des erfindungsgemäßen Mittels ist eine stabile Quervernetzung durch kovalente Bindungen, insbesondere Esterbindungen, deren Ausbildung durch mineralhaltige Säuren katalysiert wird. Kovalent verknüpfte Alginatmoleküle sind auch bereits bei Moe et al. (Food Hydrocolloids, 1991, 119) beschrieben. Allerdings nimmt das Herstellungsverfahren relativ lange Reaktionszeiten in Anspruch. Ferner sind resultierenden Produkte aufgrund der zur ihrer Herstellung verwendeten Chemikalien toxisch und somit nicht für die erfindungsgemäßen Einsatzbereiche geeignet.

Das erfindungsgemäße Mittel kann u.a. pharmazeutisch wirksame Stoffe, Nahrungsmittel bzw. Nahrungsergänzungsmittel, z.B. Vitamine, Ballaststoffe, Eiweiße, Mineralstoffe sowie andere Lebensmittelstoffe, Genußstoffe oder Aromastoffe enthalten.

Neben den genannten Stoffen können dem Trägermaterial auch weitere Hilfsstoffe beigefügt werden. Unter anderem können im Falle des Einsatzes von pharmazeutisch wirksamen Substanzen noch zusätzlich retardierende Stoffe in Frage kommen.

Außerdem können die Mittel gemäß der vorliegenden Erfindung zusätzlich Füll-, Spreng-, Binde- und Gleitmittel sowie Trägerstoffe enthalten.

In das schwammartige Gebilde können auch Wirkstoffe eingebracht werden.

Unter Wirkstoffen im Sinne der Erfindung sind alle Stoffe mit einer pharmazeutischen oder biologischen Wirkung zu verstehen. Beispiele sind Betamethason, Thioetsäure, Sotalol, Salbutamol, Norfenefrin, Solymarin, Dihydroergotamin, Buflumedil, Etofibrat, Indometacin, Oxazepam, beta-Acetyldigoxim, Piroxicam, Haloperidol, ISMN, Amitirptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-(beta-Hydroxyethyl)rutosid, Propicillin, Aciclovir-mononitrat, Paracetamol, Naftidrofuryl, Pentoxyfyllin, Propafenon, Acebutolol, L-Thyroxin, Tramadol. Bromocriptin, Loperamid, Ketotifen, Fenoterol, Ca-Dobelisat, Propranolol, Minocyclin, Nicergolin, Ambroxol, Metoprolol, beta-Sitosterin, Enalaprilhydrogenmaleat, Benzafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromirphon, Ibuprofen, Primidon, Clobazam, Oxaceprol, Medroxyprogresteron, Flecainid, Mg-Pridoxal-5-phosphatglutaminat, Hymechromon, Etofyllinclofibrat, Vincamin, Cinarizin, Diazepam, Ketoprofen, Flupentixol, Molsidomin, Glibomuid, Dimetinden, Melperon, Soquinolol, Dibydrocodein, Clomethiazol, Clemastin, Glisoxepid, Kallidinogenase, Oxyfedrin, Baclofen, Carboxymethylcystein, Thiorodacin, Betathistin, L-Tryptophan, Myrtol, Bromalaine, Prenylamin, Salazosulfapyridin, Astemizol, Sulpirid, Benzerazid, Dibenzepin, Acetylsalicylsäure, Miconazol, Nystatin, Ketonconazol, Na-Picosulfat, Colestyramin, Gemifibrocil, Rifampicin, Fluorocortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamepin, Mg-L-aspartat, Penbutolol, Piretanid, Amitriptylin, Cyproteron, Na-Valpropinat, Mebeverin, Bisacodyl, 5-Amino-Salicylsäure, Dihydralazin, Magaldrat, Phenprocoumon, Amantadin, Naproxen, Cartelol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Weitere Beispiele sind folgende Wirkstoffe: Acetaminophen (= Paracetamol), Acetohexamid, Acetyldigoxim, Acetylsalicylsäure, Acromycin, Anipamil, Benzocain, beta-Carotin, Choramphenicol, Chlordiazepoxid, Chlormadinoacetat, Chlorthiazid, Cinnarizin, Clonazepam, Codein, Decamethason, Diazepam, Dicumarol, Digitoxin, Digoxin, Dihydroergotamin, Drotaverin, Flunitrazepam, Furosemid, Gramicidin, Griseofluvin, Hexobarbital, Hydrochlorothiazid, Hydrocortison, Hydroflumethazig, Indimethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Ostradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol (= Sulfameter), Sulfaperin, Sulfathiazol, Sulfisoxazol, Testosteron, Tolazamid, Tolbutamid, Trimethoprim, Tyrothricin, Vitamine, Mineralien.

Als Wirkstoffe sind auch solche mit prophylaktischer Wirkung, beispielsweise in Bereichen der Tumortherapie, denkbar.

Neben den genannten Wirkstoffen können dem Trägermaterial auch weitere Hilfsstoffe beigefügt werden. Unter anderem können noch zusätzlich retardierende Stoffe in Frage kommen.

Als retardierende Hilfsstoffe können im Wesentlichen wasserunlösliche Hilfsstoffe oder Gemische davon, wie Lipide, u.a. Fettalkohole, z.B. Cetylalkohol, Stearylalkohol und Cetostearylalkohol; Glyceride, z.B. Glycerinmonostearat oder Gemische von Mono-, Di- und Triglyceriden pflanzlicher Öle; hydrierte Öle, wie hydriertes Rizinusöl oder hydriertes Baumwollsamenöl; Wachse, z.B. Bienenwachs oder Carnaubawachs; feste Kohlenwasserstoffe, z.B. Paraffin oder Erdwachs; Fettsäuren, z.B. Stearinsäure; gewisse Cellulosederivate, z.B. Ethylcellulose oder Acetylcellulose; Polymere oder Copolymere, wie Polyalkylene, z.B. Polyäthylen, Polyvinylverbindungen, z.B.. Polyvinylchlorid oder Polyvinylacetat, sowie Vinylchlorid-Vinylacetat-Copolymere und Copolymere mit Crotonsäure, oder Polymere und Copolymere von Acrylaten und Methacrylaten, z.B. Copolymerisate von Acrylsäureester und Methacrylsäuremethylester, verwendet werden.

Das resultierende, in Wasser und/oder gastrointestinalen Flüssigkeiten schwer lösliche bzw. gering resorbierbare Material kann anschließend komprimiert werden. Dies kann durch Pressen, Walzen oder vergleichbare Methoden geschehen. Ferner kann eine Komprimierung des Materials durch Kaubewegungen bei der oralen Einnahme des Materials erfolgen.

Vor, während oder nach der Herstellung des schwammartigen Gebildes kann das Material beispielsweise mit den oben erwähnten wirksamen Stoffen beaufschlagt werden. Hierfür kommen alle üblichen Methoden in Betracht. Im einfachsten Falle kann dies während der Herstellungsphase des Schwammaterials durch Mischen von Trägermaterial und wirksamem Stoff erfolgen. Ebenso können diese Stoffe auf die Oberfläche aufgebracht werden.

Das so hergestellte schwammartige Gebilde kann in einer bevorzugten Ausführungsform der Erfindung mit den zuvor erwähnten Stoffen umhüllt werden. D.h., entweder wird aus dem Stoff ein Behältnis, z.B. eine Kapselhülle, hergestellt und in diese das schwammartige Gebilde eingebracht. Oder auf das Gebilde wird der Stoff direkt aufgebracht, etwa durch Tauchen, Besprühen, Aufstreichen oder ähnliche Methoden. In einer anderen Ausführungsform der Erfindung wird das schwammartige Gebilde in den Stoff eingebracht. Dies kann z.B. durch Tränken erreicht werden.

Zweck des erfingungsgemäßen Verfahrens ist es, ein Mittel zu erhalten, daß bei Durchtritt durch die Speiseröhre ausreichend komprimiert ist und sich erst im Magen dekomprimiert. Dieses Ziel wird mit den genannten Verfahrensschritten erreicht.

Im Gegensatz zu anderen Lebensmittel-/ Nahrungsergänzungs-/ Diät- oder Arzneimittelprodukten, die kurzfristig im Magen zersetzt werden oder schon in zerkleinertem Zustand in diesen gelangen, behält der aus natürlichen, halbsynthetischen oder synthetischen Polymeren bestehende, in der beschriebenen Weise hergestellte Schwamm- oder Schaumkörper durch besondere Vernetzungsstellen, insbesondere kovalente Bindungen über mehrere Stunden seine ursprünglich vorhandene Form. Durch die Dekomprimierung des erfindungsgemäßen Mittels im Magen erfolgt eine Anregung der Dehnungsrezeptoren des Magens, die ein Sättigungsgefühl auslöst. Der erfindungsgemäße Schwamm wird dabei im Magen nur schwer aufgelöst bzw. nur geringfügig resorbiert.

Ferner bezieht sich die vorliegende Erfindung auf ein Verfahren zur Herstellung von Mitteln zur Erzeugung eines langanhaltenden Sättigungseffektes. Dabei werden Polyuronsäure-haltige Polysaccharide über ionische Bindungen vernetzt, eingefroren, gefriergetrocknet, über kovalente Bindungen stabil quervernetzt, anschließend getrocknet und gegebenenfalls gepreßt. Besonders bevorzugt werden hier als lineare Polyuronsäure-haltige Polysaccharide Alginsäuren und deren Salze eingesetzt. Darüber hinaus sind auch Pectine, Xanthan, Tragant, Chondroitinsulfat sowie alle anderen Uronsäure-haltigen Verbindungen oder deren Salze denkbar.

Erfindungsgemäß werden Alginsäuren oder deren Salze in Konzentrationen von 0,3 bis 10 Gew.-% vorzugsweise 0,5 bis 5 Gew.-%, besonders bevorzugt in Konzentrationen von 1 bis 3 Gew.-% eingesetzt.

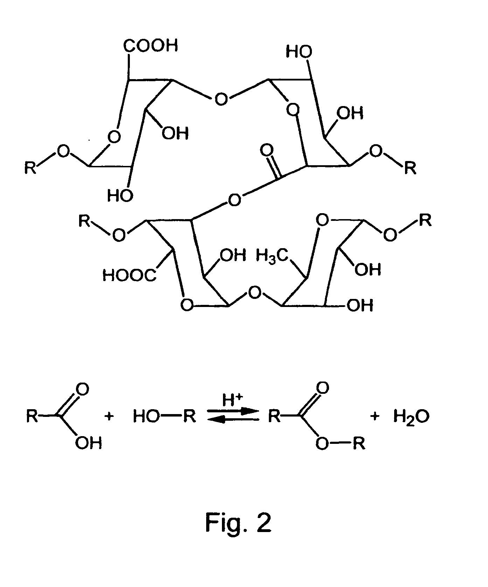

Erfindungswesentlich ist ferner, daß durch Eintauchen der schwammartigen Gebilde in mineralhaltige Säuren, vorzugsweise Salzsäure, im Anschluß an die Gefriertrocknung zusätzliche, stabile Vernetzungsstellen durch die Ausbildung von kovalenten Esterbindungen in das Schwammaterial eingeführt werden (Fig. 2). Hierbei werden nach Ermessen des Fachmannes wenigstens katalytische Mengen an mineralhaltigen Säuren eingesetzt, höchstens jedoch eine so große Menge, daß das Material nicht durch eine saure Hydrolyse in seine Bestandteile aufgelöst wird. Besonders bevorzugt ist eine Konzentration von 0,1 mol/l mineralhaltige Säure, insbesondere Salzsäure. Die stabile Quervernetzung durch mineralhaltige Säuren bewirkt eine lang andauernde Schwerlöslichkeit des Schwammkörpers in Wasser und/oder gastrointestinalen Flüssigkeiten.. Diese Schwerlöslichkeit ist Voraussetzung für den langen Aufenthalt des Schwammes im Magen und den dadurch bewirkten anhaltenden Sättigungseffekt.

Die Erfindung ist nicht auf das beschriebene Verfahren beschränkt, sondern gilt auch für alle anderen Verfahren, bei denen Schwämme oder schwammähnlichen Gebilde hergestellt werden, die durch eine Schwerlöslichkeit in Wasser und/oder gastrointestinalen Flüssigkeiten und die sich dadurch ergebende lange Verweildauer im Magen einen langfristigen Sättigungseffekt erzielen sollen oder können.

Das erfindungsgemäße Mittel wird oral eingenommen. Der feste Schwamm- oder feste Schaumkörper passiert durch Hinzufügen von Trinkflüssigkeit sowie leichte Kau- oder Schluckbewegungen Mund, Rachen und Speiseröhre und schwemmt durch die Magenflüssigkeit vorzugsweise zu seinem ursprünglichen Volumen im Magen wieder auf. Gegebenenfalls kann das Volumen auch größer oder kleiner als das ursprüngliche sein.

Durch die orale Einnahme des erfindungsgemäßen Mittels wird erreicht, daß der feste Schwamm- oder feste Schaumkörper durch die Schwerlöslichkeit im Magen über mehrere Stunden im Magen verweilt. Infolgedessen läßt sich ein langfristiges Sättigungs- oder Völlegefühl erzielen, das eine reduzierte Nahrungsaufnahme zur Folge hat. Ebenso kann das Mittel aber auch in den Bereichen der Pharmazie und/oder des Gesundheitswesens, bevorzugt der (Diät-)Ernährung oder Nahrungsergänzung zum Einsatz kommen. Zu diesem Zweck enthält das Mittel die bereits oben beschriebenen Wirkstoffe oder Nahrungsmittel.

Je nach gewünschtem Sättigungsgrad, kann eine unterschiedliche Anzahl an Schwammkörper in unterschiedlichen Zeitabständen täglich eingenommen werden. Die durch das im Magen befindliche Schwammvolumen angesprochenen "Dehnungsrezeptoren" erzeugen über das Zwischenhim einen Sättigungseffekt, der erst bei Leerung des Magens wieder zurückgeht. Somit kann durch die Länge des Aufenthaltes der Volumenschwämme die Sättigungsdauer gesteuert werden.

Ferner bezieht sich die vorliegende Erfindung auf die Verwendung der erfindungsgemäßen Mittel zur Herstellung von Mitteln zur Erzeugung eines Sättigungseffekts sowie zur Herstellung von oral verabreichbaren Arzneimitteln, mit Wirkstoffen beaufschlagten Nahrungsmitteln, Nahrungsergänzungsmitteln oder Diätnahrungsmitteln.

Ferner können die erfindungsgemäßen Mittel auch nach Durchschritt durch den Magen, also im Darm, ihre Wirkung entfalten. Hier wirkt das Mittel durch die Anregung der Dehnungsrezeptoren in der Darmwand insbesondere stimulierend auf die Darmtätigkeit.

In einer besonderen Ausführungsvariante der Erfindung kann das Mittel auch so ausgestaltet sein, daß die Dekomprimierung erst im Darm erfolgt. D.h., das Mittel entfaltet in diesem Fall seine Wirkung nicht im Magen, sondern nur im Darm. Zu diesem Zweck ist vorzugsweise vorgesehen, die Polymere mit einer Verbindung zu versehen, die sich nicht im Magen, sondern erst im Darm auflöst, so daß sich das komprimierte schwammartige Gebilde auch erst dort zu dekomprimieren vermag.

Die Auflösung der Verbindung wird dabei durch verschiedene, z. T. auch gleichzeitig im Darm vorherrschende Parameter beeinflußt, wie z. B. pH-Wert, Druck, Redoxpotential und enzymatische Auflösung durch die Darmflora. Darüber hinaus beeinflußt auch die Verweitzeit des Mittels im Darm die Geschwindigkeit mit der sich die Verbindung auflöst.

Vorzugsweise löst sich die Verbindung bei einem pH-Wert zwischen 5 und 10, bevorzugt zwischen 7 und 9, besonders bevorzugt zwischen 5,5 und 8,5 auf. Höchst bevorzugt ist eine Auflösung im pH-Milieu des Darms bei einem pH-Wert zwischen 6,4 ± 0,6 bis 7,0 ± 0,7. Es eignen sich insbesondere solche Verbindungen, die sich in Abhängigkeit von dem Redoxpotential, enzymatischer Aktivitäten und Druck auflösen.

Die Verbindung wird erfindungsgemäß bevorzugt in Form einer Beschichtung auf das schwammartige Gebilde aufgebracht, die gegebenenfalls auch aus mehreren Schichten aufgebaut sein kann. Die Mindestschichtdicke kann dabei erheblich variieren und ist abhängig von dem verwendeten Filmbildner und seiner Zusammensetzung. Osterwald H. et al. (Acta Pharm Technol, 1980, 26: 201-209) beschreibt beispielsweise eine Mindestschichtdicke von 46 µm für die Zubereitung eines Filmbildners in organischen Lösungsmitteln, mit Ammoniumsalzlösung zubereitet sind 161 µm Schichtdicke erforderlich, als Emulsion 46 µm und als Latexdispersion 52 µm Schichtdicke. Erfindungsgemäß liegt die Schichtdicke zwischen 10 µm bis mehrere Millimeter, bevorzugt zwischen 15 µm bis 3 mm.

Anstelle einer direkt auf das Gebilde aufgebrachten Beschichtung kann das schwammartige Gebilde jedoch auch in ein Behältnis eingebracht werden, das sich unter den oben beschriebenen Bedingungen auflöst. D.h., das Behältnis ist im Magen beständig, während es sich im Darm auflöst.

In einer anderen Variante der Erfindung kann die Verbindung in das schwammartige Gebilde eingebracht sein. Dies läßt sich beispielsweise durch Tränken in einer Lösung der Verbindung erreichen oder durch Beimischen der Verbindung während der Herstellung des schwammartigen Gebildes. Selbstverständlich kann ein derart, beispielsweise getränktes Gebilde zusätzlich auch mit einer Beschichtung der Verbindung versehen sein. Ebenso kann das getränkte Gebilde auch in das oben beschriebene Behältnis eingebracht sein. Ferner kann das Gebilde in ein Behältnis eingebracht sein, das seinerseits mit der Verbindung beschichtet oder durchtränkt ist oder in das die Verbindung eingebracht ist.

Die zeitliche und lokale Auflösung der Verbindung läßt sich durch die Auswahl und Kombination der Verbindungen beeinflussen, wodurch eine gezielte Freisetzung des schwammartigen Gebildes im Darm und insbesondere in den verschiedenen Darmabschnitten, wie Jejunum, Ileum und Kolon, erreicht wird. Die Löslichkeit der Verbindungen kann dabei von einem oder mehreren Faktoren abhängig sein, wie beispielsweise pH-Wert, Einwirkzeit, Redoxpotential des Darmes, enzymatische Aktivitäten der Därmflora oder Druck, der durch die intestinale Peristaltik erzeugt wird. Die verschiedenen Möglichkeiten zur Steuerung der Freisetzung von Wirkstoffen sind zahlreich beschrieben. Die pH-abhängige Löslichkeit wird beispielsweise bei *Marvola et al.,* Eur J Pharm Sci, 1999, 7:259-267 und *Khan ZI et al.,* J Controlled Release, 1999, 58:215-222 beschrieben. *Pozzi F. et al.,* J Controlled Release, 1994, 31:99-108; *Wilding IR et al.,* Pharmacol Ther, 1994, 62:97-124; *Niwa K. et al.,* J Drug Target, 1995, 3:83-89 und US-4871549 offenbaren Systeme, die die Wirkstoffe in Abhängigkeit von der Zeit freisetzen. Beispiele für Systeme mit einer kombinierten pH-Wert und Zeitabhängigkeit sind in *Rodriguez M. et al.,* J Controlled Release, 1998, 55:67-77 und *Gazzinga A. et al.,* STP Pharm Sci, 1995, 5:83-88 beschrieben. Mit der Auflösung von Verbindungen, bedingt durch ein verändertes Redoxpotential im Darm, beschäftigen sich *Bronsted H. et al.,* Pharm Res 1992, 9:1540-1545; *Yeh PY et al.*, J Controlled Release, 1995, 36: 109-124; *Shanta KL et al.,* Biomaterials, 1995, 16:1313-1318 und *Kimura Y et al.,* Polymer, 1992, 33: 5294-5299. Beispiele für Systeme, die durch die Enzyme der Darmflora freigesetzt werden, sind in *Ashford M et al.,* J Controlled Release, 1994, 30:225-232; *Fernandez-Hervas MJ et al.,* Int J Pharm, 1998, 169:115-119; *EP-0460921; US-4432966* und *Milojevic S et al.,* J Controlled Release, 1996, 38:75-84; beschrieben. Die Auflösung von Systemen durch den Druck der intestinalen Peristaltik wird in *Muraoka M et al.,* J Controlled Release, 1998, 52:119-129 behandelt.

Erfindungsgemäß bevorzugt werden dabei folgende Verbindungen und deren Kombinationen, die jedoch keineswegs limitierend für die vorliegende Erfindung sind:

Hydroxypropyl-methylcellulose-phthalat (HPMCP 55), Hydroxypropyl-methylcellulose-acetat-succinat (Aqoat AS-MF. Aqoat AS-HF), 1:1 Kopolymer aus Methacrylsäure und Ethylacrylat (Eudragit®L), Kopolymer aus Vinylacetat und Crotonsäure (Coating CE 5142), Cellulose-acetatphthalat (CAP, Aquateric), Methacrylat-Kopolymere (Eudragit®S), Schellack, Time Clock System®, Carnaubawachs, Hydroxypropylmethylcellulose (TC-5), Pulsincap®, Polyethylenglykol, vernetztes Polyethylenglykol, Ethylcellulose, Ethylcellulose-Ethanol-Gemisch, Hydroxypropylcellulose, Hydroxypropylmethylcellulose, GlycerinMonostearat, Eudragit®E. Ebenso sind Hydrogele aus Azo-Verbindungen möglich, wie beispielsweise N-substituiertes Methacrylamid, N-tert-butylacrylamid, Acrylsäure in Gegenwart von 4,4'-Bis-(methacryloylamino)-azobenzen,4,4'-Bis(N-methacryloyl-6-aminohexanoylamino)azobenzen oder 3,3',5,5'-Tetrabromo-4,4,4',4'-tetra(methacryloylamino)azobenzen. Beispiele für weitere Verbindungen sind lineare Polymervorstufen, beispielsweise enthaltend N,N-Dimethylacrylamid, N-tert-Butylacrylamid, Acrylsäure, N-Methacryloyl-glycyl-glycin-p-nitrophenylester, quervemetzt durch geeignete Vernetzter, wie z.B. N,N'-(ω-aminocaproyl)-4,4'-diaminoazobenzen sowie Polymere enthaltend Azoverbindungen, wie beispielsweise 2-Hydroxy-ethyl-Methacrylat, 4-(Methacryloyloxy)azobenzen, N-(2-hydroxypropyl)methacrylamid-Kopolymere, Kopolymere enthaltend Styrol und 2-Hydroxyethylmethacrylat vernetzt durch beispielsweise 4,4'-Divinylazobenzen oder N,N'-Bis-(β-sterylsulfonyl)-4,4'-diaminoazobenzen. Ebenso sind erfindungsgemäß Poly(ether-ester)azo-Polymere einsetzbar, wie beispielsweise Kopolymere enthaltend 4-[4-[(6-hydroxyhexyl)-oxy]phenyl]azobenzoesäure und 16-Hydroxyhexadecansäure, Kopolymere enthaltend 4-[2-[2-(2-hydroxyethoxy)ethoxy]ethoxy]benzoesäure, 4-[4-[2-[2-(2-hydroxyethoxy)ethoxy]ethoxy]phenyl]azobenzoesäure und 16-Hydroxyhexadecansäure oder 12-Hydroxydodecansäure sowie segmentierte Polyurethane enthaltend m-Xylen-Diisocyanat, 3,3'-Dihydroxyazobenzen, Polyethylenglycol oder 1,2-Propandiol. Ferner einsetzbar sind Azoverbindungen enthaltende Polyamide oder Kopolymere aus 4-[4-(chlorocarbonyl)phenyl)]-azobenzoylchlorid und α,ω-Bis(aminopropyl)-poly(tetramethylenoxid) sowie Kopolymere aus 4-[4-(Chlorocarbonyl)phenyl]azobenzoylchlorid und Jeffamine ED-600.
Ferner finden Pektine Verwendung, die zusätzlich ummantelt oder in einer Matrix eingebettet werden können, wie beispielsweise Methoxy-Pektin, amidiertes Pektin, Calciumpektinat, Pektin in Kombination mit Ethylcellulose (Aquacoat, Surelease), Acrylsäureester-Polymere (Eudragit RS30D, Eudragit NE30D). Ebenso finden Kombinationen von Pektinen mit anderen Ballaststoffen Einsatz. Beispiele für Ballaststoffe sind Guar (Galactomannan) oder Chitosan, wobei die Ballaststoffe selbst wiederum ummantelt oder Bestandteil einer Matrix sein können. Hierbei finden folgende Substanzen Einsatz als Filmbildner: Polymethacrylatlösungen, Kopolymerisate enthaltend Polyurethan und Di-, Oligo- oder Polysaccharide (Galactomannane) sowie Ethylgalactomannane oder Acetylgalactomannane. Ebenso finden Cyanoacrylat, Inulin, Inulin-Suspensionen mit Eudragit-RS, methacryliertes Inulin, Chondroitinsulfat, Chondroitin-Polymere enthaltend 1,12-Diaminododecan und Dicyclohexylcarbodiimid, amorphe Amylose oder amorphe Amylose zusammen mit anderen filmbildenden Polymeren als Filmbildner Einsatz. Ebenso können Dextrane verwendet werden, die verschiedenartig vernetzt sein können, beispielsweise mit Diisocyanaten, Fettsäureestern, beispielsweise der Laurylsäure, Glutaraldehyd. Auch Konjugate aus Biphenylessigsäure und β-Cyclodextrin, Filme aus β-Cyclodextrinen mit Methacrylsäure-Kopolymeren oder Acrylsäurepolymere mit Disaccharidseitengruppen kommen erfindungsgemäß zum Einsatz.

Die Auswahl der Verbindungen sowie deren vielfältige Kombinationsmöglichkeiten ermöglichen eine gezielte Freisetzung des schwammartigen Gebildes im Dickdarm.

Im Folgenden wird die Erfindung mit Bezugnahme auf das folgende Beispiel näher erläutert:

### Herstellung von Alginatschwämmen:

In die Vertiefungen einer Mikrotiterplatte (Durchmesser 16 mm, Höhe 20 mm) werden jeweils 0,5 ml einer 1%igen Natriumalginatlösung (w/v) pipettiert und mit jeweils 0,5 ml destilliertem Wasser sowie unter intensivem Rühren mit einer 0,2%igen Calciumgluconatlösung (w/v) versetzt. Die so erzeugten Hydrogele werden bei -20 °C über Nacht eingefroren und anschließend bei 0,007 mm Hg (Quecksilbersäule) und -60 °C gefriergetrocknet.

Die gefriergetrockneten Schwämmchen werden aus der Mikrotiterplatte genommen und für 30 Sekunden in 0,1 molarer Salzsäure getaucht. Die Entfernung der Salzsäure erfolgt durch Spülen mit destilliertem Wasser. Die Schwämmchen werden bei 30 °C im Trockenschrank getrocknet und anschließend verpreßt.

## Patentansprüche

1. Mittel zur oralen Einnahme, enthaltend stabil miteinander vernetzte Uronsäure-haltige Polysaccharide in Form eines schwammartigen Gebildes, das in Wasser und/oder gastrointestinalen Flüssigkeiten schwer löslich bzw. gering resorbierbar ist, **dadurch gekennzeichnet, dass** die Uronsäure-haltigen Polysaccharide durch ionische Bindungen miteinander vernetzt und zusätzlich durch kovalente Bindungen stabil miteinander quervemetzt sind.

2. Mittel nach einem des Anspruchs 1, **dadurch gekennzeichnet, daß** die Uronsäure-haltigen Polysaccharide Alginsäuren, Pectine, Xanthan, Tragant, Chondroitinsulfat sowie alle Uronsäure-haltigen Verbindungen oder deren Salze sind.

3. Mittel nach einem der Ansprüche 1 oder 2, **dadurch gekennzeichnet, daß** es als Quervernetzung kovalente Bindungen, vorzugsweise durch mineralhaltige Säuren katalysierte Esterbindungen, enthält.

4. Mittel nach einem der Ansprüche 1 bis 3, **dadurch gekennzeichnet, daß** Wirkstoffe in/auf das schwammartige Gebilde ein-/aufgebracht sind oder das schwammartige Gebilde umhüllen.

5. Verfahren zur Herstellung von Mitteln gemäß einem der vorhergehenden Ansprüche, **dadurch gekennzeichnet, daß** Polyuronsäure-haltige Polysaccharide
a) über ionische Bindungen vernetzt,
b) eingefroren,
c) gefriergetrocknet,
d) über kovalente Bindungen stabil quervemetzt und
e) anschließend getrocknet und
f) gegebenenfalls gepreßt
werden.

6. Verfahren nach Anspruch 5, **dadurch gekennzeichnet, daß** als lineare Polyuronsäure-haltige Polysaccharide Alginsäuren, Pectine, Xanthan, Tragant, Chondroitinsulfat sowie alle Uronsäure-haltigen Verbindungen oder deren Salze eingesetzt werden.

7. Verfahren nach einem der Ansprüche 5 oder 6, **dadurch gekennzeichnet, daß** Alginsäuren oder deren Salze in Konzentrationen von 0,3 bis 10 Gew.-% vorzugsweise 0,5 bis 5 Gew.-%, besonders bevorzugt von 1 bis 3 Gew.-% eingesetzt werden.

8. Verfahren nach einem der Ansprüche 5 bis 7, **dadurch gekennzeichnet, daß** die mineralhaltigen Säuren bevorzugt in einer Konzentration von 0,1 mol/l eingesetzt werden.

9. Verfahren nach einem der Ansprüche 5 bis 8, **dadurch gekennzeichnet, daß** als Mineralsalzlösung Salzsäurelösung verwendet wird.

10. Verwendung der Mittel gemäß einem der Ansprüche 1 bis 4 zur Herstellung von Mitteln zur Erzeugung eines Sättigungseffekts und/oder zur Verbesserung der Darmtätigkeit.

11. Verwendung der Mittel gemäß einem der Ansprüche 1 bis 4 zur Herstellung von oral verabreichbaren Arzneimitteln, mit Wirkstoffen beaufschlagten Nahrungsrnitteln, Nahrungsergänzungsmitteln oder Diätnahrungsmitteln.

## Claims

1. Composition for oral intake comprising uronic-acid-containing polysaccharides stably crosslinked to one another in the form of a sponge-like structure which is only slightly soluble or of low absorbability in water and/or gastrointestinal fluids, **characterized in that** the uronic-acid-containing polysaccharides are crosslinked to one another by ionic bonds and in addition are stably crosslinked to one another by covalent bonds.

2. Composition according to Claim 1, **characterized in that** the uronic-acid-containing polysaccharides are alginic acids, pectins, xanthan, tragacanth, chondroitin sulphate and all uronic-acid-containing compounds or their salts.

3. Composition according to one of Claims 1 or 2, **characterized in that** it contains, as crosslinking, covalent bonds, preferably ester bonds catalyzed by mineral acids.

4. Composition according to one of Claims 1 to 3, **characterized in that** active compounds are introduced into/applied onto the sponge-like structure or encase the sponge-like structure.

5. Process for preparing compositions according to one of the preceding claims, **characterized in that** polyuronic-acid-containing polysaccharides
a) are crosslinked via ionic bonds,
b) are frozen,
c) are freeze-dried,
d) are stably crosslinked via covalent bonds and
e) are then dried and
f) are, if appropriate, pressed.

6. Process according to Claim 5, **characterized in that** the unbranched polyuronic-acid-containing polysaccharides used are alginic acids, pectins, xanthan, tragacanth, chondroitin sulphate and all uronic-acid-containing compounds or their salts.

7. Process according to one of Claims 5 or 6, **characterized in that** alginic acids or their salts are used in concentrations of 0.3 to 10% by weight, preferably 0.5 to 5% by weight, particularly preferably from 1 to 3% by weight.

8. Process according to one of Claims 5 to 7, **characterized in that** the mineral acids are preferably used in a concentration of 0.1 mol/l.

9. Process according to one of Claims 5 to 8, **characterized in that** the mineral salt solution used is hydrochloric acid solution.

10. Use of the composition according to one of Claims 1 to 4 for preparing compositions for producing a satiation effect and/or for improving intestinal activity.

11. Use of the composition according to one of Claims 1 to 4 for preparing drugs which can be administered orally, foodstuffs which are loaded with active compounds, food supplements, or dietetic foods.

## Revendications

1. Agent destiné à l'ingestion orale, qui contient des polysaccharides contenant de l'acide uronique et réticulés de manière stable les uns avec les autres pour former un produit de forme spongieuse difficilement soluble ou peu résorbable dans l'eau et/ou dans les liquides gastro-intestinaux, **caractérisé en ce que** les polysaccharides contenant de l'acide uronique sont réticulés les uns avec les autres par des liaisons ioniques et sont de plus réticulés transversalement les uns avec les autres de manière stable par des liaisons covalentes.

2. Agent selon la revendication 1, **caractérisé en ce que** les polysaccharides qui contiennent de l'acide uronique sont des acides alginiques, des pectines, du xanthane, de l'adragante, du sulfate de chondroïtine ainsi que tous les composés qui contiennent de l'acide uronique, ou des sels de ces composés.

3. Agent selon la revendication 1 ou 2, **caractérisé en ce que** pour la réticulation transversale, il contient des liaisons covalentes et de préférence des liaisons ester catalysées par des acides qui contiennent des minéraux.

4. Agent selon l'une quelconque des revendications 1 à 3, **caractérisé en ce que** des substances actives sont incorporées ou appliquées dans ou sur le produit spongieux ou entourent le produit spongieux.

5. Procédé de fabrication d'agents selon l'une quelconque des revendications précédentes, **caractérisé en ce que** les polysaccharides qui contiennent de l'acide polyuronique sont
a) réticulés par l'intermédiaire de liaisons ioniques,
b) congelés,
c) lyophilisés,
d) réticulés transversalement de manière stable par des liaisons covalentes et
e) ensuite séchés et
f) éventuellement comprimés.

6. Procédé selon la revendication 5, **caractérisé en ce que** comme polysaccharides linéaires qui contiennent de l'acide polyuronique, on utilise des acides alginiques, des pectines, du xanthane, de l'adragante, du sulfate de chondroïtine ainsi que tous les composés qui contiennent de l'acide uronique, ou des sels de ces composés.

7. Procédé selon la revendication 5 ou 6, **caractérisé en ce que** les acides alginiques ou leurs sels sont utilisés à des concentrations de 0,3 à 10% en poids, de préférence de 0,5 à 5% en poids et de façon particulièrement préférable de 1 à 3% en poids.

8. Procédé selon l'une quelconque des revendications 5 à 7, **caractérisé en ce que** les acides contenant des minéraux sont utilisés de préférence à une concentration de 0,1 mole/l.

9. Procédé selon l'une quelconque des revendications 5 à 8, **caractérisé en ce que** comme solution de sels minéraux, on utilise une solution d'acide chlorhydrique.

10. Utilisation des agents selon l'une quelconque des revendications 1 à 4 pour la préparation d'agents qui créent un effet rassasiant et/ou pour améliorer l'activité de l'intestin.

11. Utilisation des agents selon l'une quelconque des revendications 1 à 4 pour la préparation de médicaments administrés oralement, d'aliments complétés de substances actives, d'agents de complémentation alimentaire ou d'aliments de régime.
